# EUROPEAN PATENT APPLICATION

(11) **EP 2 352 033 A2**
(43) Date of publication of application: **03.08.2011**
(21) Application number: 11152494.8
(22) Date of filing: 28.01.2011
(51) Int. Cl.: G01N 35/00, G06F 19/00

(54) **Sample analyzer, sample analyzing method and computer program product**

(30) Priority: 28.01.2010 JP 2010016436
(71) Applicant: SYSMEX CORPORATION, Kobe-shi, Hyogo 651-0073 (JP)
(72) Inventor: Tanoshima, Eiji, Hyogo 651-0073 (JP); Hama, Eiji, Nagano 399-0702 (JP)
(74) Representative: HOFFMANN EITLE

(57) **Abstract**

A sample analyzer comprising: a measurement section for performing measurement of a sample; a display for displaying an analysis result that is obtained based on the measurement of the sample performed by the measurement section; a controller for receiving a transmission instruction to transmit a screen displayed by the display; and a transmitter for transmitting, to an external destination, image data of the screen for which the controller has received the transmission instruction, together with identification information for identifying the sample analyzer or a facility where the sample analyzer is installed. A method for controlling a sample analyzer and a computer program product are also disclosed.

## Description

### FIELD OF THE INVENTION

The present invention relates to a sample analyzer for performing sample analysis. The present invention also relates to a sample analyzing method and a computer program product.

### BACKGROUND

There are known techniques in which a sample analyzer for performing sample analysis is connected via a communication network to a management server that is installed in a facility of a maintenance service provider. It is also known that such a sample analyzer generates a composite image by combining a displayed image and a template image and transmits the generated composite image to the management server in accordance with a transmission instruction given by a user (see JP laid-open patent application 2008-20309, for example).

When the management server receives the composite image, the maintenance service provider, which is the user of the management server, identifies the sample analyzer which has transmitted the composite image or a facility where the sample analyzer is installed. Then, the maintenance service provider contacts a person in charge of the sample analyzer in the facility. However, JP laid-open patent application 2008-20309 gives no description regarding the manner of identifying the sample analyzer which is the source of the transmitted image or the facility where the sample analyzer is installed. Therefore, based on the conventional technique, it is difficult for the maintenance service provider to promptly contact the person in charge of the sample analyzer in the facility. Thus, the conventional technique has a problem in that it is difficult for the user of the sample analyzer to receive a maintenance service promptly.

Moreover, JP laid-open patent application 2008-20309 does not give any description about transmitting an image for the purpose of receiving academic advice.

The present invention has been devised in view of the above. A main object of the present invention is to provide a sample analyzer that makes it possible to promptly receive a maintenance service or academic advice.

### SUMMARY OF THE INVENTION

A first aspect of the present invention is a sample analyzer comprising: a measurement section for performing measurement of a sample; a display for displaying an analysis result that is obtained based on the measurement of the sample performed by the measurement section; a controller for receiving a transmission instruction to transmit a screen displayed by the display; and a transmitter for transmitting, to an external destination, image data of the screen for which the controller has received the transmission instruction, together with identification information for identifying the sample analyzer or a facility where the sample analyzer is installed.

In the aforementioned sample analyzer, it is desirable that the transmitter is configured to transmit an email to which the image data is attached.

In the aforementioned sample analyzer, it is desirable that the identification information is transmitted as a part of source information which indicates the source of the email.

In the aforementioned sample analyzer, it is desirable that the subject of the email contains the model name of the sample analyzer.

In the aforementioned sample analyzer, it is desirable that the sample analyzer does not have a function of receiving an incoming email.

In the aforementioned sample analyzer, it is desirable that the identification information contains an address for linking to information that contains contact information about the facility where the sample analyzer is installed.

In the aforementioned sample analyzer, it is desirable that the controller is configured to obtain status information about a status of the sample analyzer, and
the transmitter is configured to transmit the status information together with the image data and the identification information.

In the aforementioned sample analyzer, it is desirable that the display comprises a touch panel type input section, and the controller is configured to receive the transmission instruction, which is provided via the input section.

In the aforementioned sample analyzer, it is desirable that the controller is configured to cause, if the controller has received a predetermined input via the input section while a screen to be transmitted is displayed by the display, the display to switch the displayed screen to a reception screen for receiving the transmission instruction, and to receive the transmission instruction via the reception screen.

In the aforementioned sample analyzer, it is desirable that the reception screen includes a button for receiving the transmission instruction and a button for receiving an instruction different from the transmission instruction.

In the aforementioned sample analyzer, it is desirable that the controller is configured to display a button for receiving the predetermined input, at a predetermined position on the display regardless of contents of the displayed screen.

In the aforementioned sample analyzer, it is desirable that the controller is configured to store image data of the screen to be transmitted if the controller has received the predetermined input via the input section while the screen to be transmitted is displayed by the display, and to cause the display to switch the displayed screen to the reception screen for receiving the transmission instruction, and the transmitter is configured to transmit the image data stored in the controller when the controller has received the transmission instruction.

In the aforementioned sample analyzer, it is desirable that the controller is configured to delete, after receiving the predetermined input via the input section and storing the image data, the image data stored in the controller and store image data of another screen if the controller has received the predetermined input via the input section again while the other screen is displayed by the display.

A second aspect of the present invention is a method for controlling a sample analyzer which comprises a measurement section for performing measurement of a sample, a display for displaying an analysis result that is obtained based on the measurement of the sample performed by the measurement section, a transmitter for transmitting information to an external destination, and a controller, the method comprising: receiving, by the controller, a transmission instruction to transmit a screen displayed by the display; and transmitting, to an external destination by the transmitter, image data of the screen for which the controller has received the transmission instruction, together with identification information for identifying the sample analyzer or a facility where the sample analyzer is installed.

A third aspect of the present invention is a computer program product executable by a controller of a sample analyzer which comprises a measurement section for performing measurement of a sample, a display for displaying an analysis result that is obtained based on the measurement of the sample performed by the measurement section, a transmitter for transmitting information to an external destination, and the controller, the computer program product comprising: a computer readable medium for storing instructions enabling a processor to carry out operations comprising: receiving, by the controller, a transmission instruction to transmit a screen displayed by the display; and transmitting, to an external destination by the transmitter, image data of the screen for which the controller has received the transmission instruction, together with identification information for identifying the sample analyzer or a facility where the sample analyzer is installed.

### BRIEF DESCRIPTION OF THE DRAWINGS

FIG. 1 is a perspective view showing an external view of a sample analyzer according to an embodiment of the present invention;
FIG. 2 is an enlarged perspective view showing a part of the sample analyzer according to the embodiment;
FIG. 3 shows an example of an analysis result screen;
FIG. 4 shows an example of a quality control screen;
FIG. 5 shows an example of a calibration curve screen;
FIG. 6 is a schematic diagram briefly showing a maintenance service system;
FIG. 7 is a block diagram showing a part of the configuration of the sample analyzer according to the embodiment;
FIG. 8 is a block diagram showing functions of a communication program;
FIG. 9 is a flowchart showing a sequence of processing which a controller and a communication section of the sample analyzer according to the embodiment perform when the sample analyzer performs a maintenance service requesting operation;
FIG. 10 shows a maintenance menu screen;
FIG. 11 shows an email transmission notification screen;
FIG. 12 shows a transmission completion notification screen;
FIG. 13 shows a transmission failure notification screen;
FIG. 14 shows an example of an email, for requesting a maintenance service or academic advice, which is displayed on a display of a client computer;
FIG. 15 shows another example of an email, for requesting a maintenance service or academic advice, which is displayed on the display of the client computer; and
FIG. 16 shows an example of facility information displayed by the client computer.

### DESCRIPTION OF THE PREFERRED EMBODIMENTS

Hereinafter, a preferred embodiment of the present invention will be described with reference to the accompanying drawings.

### [Configuration of Sample Analyzer]

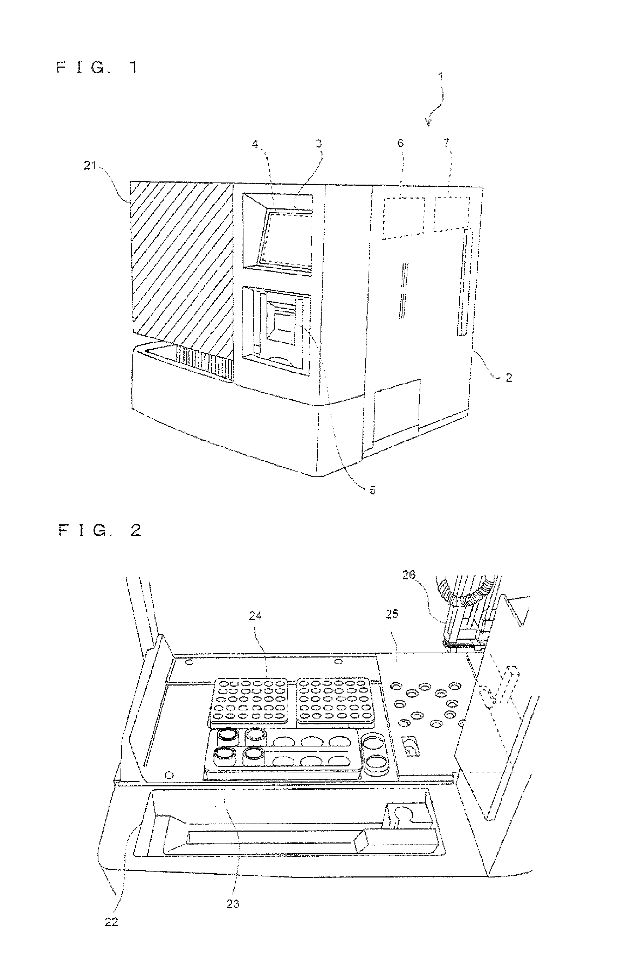

FIG. 1 is a perspective view showing an external view of a sample analyzer according to the present embodiment. A sample analyzer 1 according to the present embodiment is a blood coagulation measuring apparatus for analyzing activation of coagulation factors in a blood sample. As shown in FIG. 1, the sample analyzer 1 is structured as a built-in type apparatus, which is substantially a parallelepiped. The sample analyzer 1 includes a casing 2 for holding respective components of the sample analyzer 1. The upper left part of the casing 2 is recessed. A cover 21, which is openable and closable, covers the recess. A display 3, which includes a liquid crystal panel, is provided at the right side of the front face of the casing 2. The display 3 is structured as a touch panel type display. The display 3 includes a touch panel 4 (i.e., an input section) installed at the front face thereof. A printer 5 for printing out analysis results is provided below the display 3. A controller 6 for controlling the respective components of the sample analyzer 1 and a communication section 7 for communicating with external apparatuses are provided within the casing 2.

FIG. 2 is an enlarged perspective view showing a part of the sample analyzer 1 of which the cover 21 is opened. As shown in FIG. 2, a sample container holder 22 for placing therein sample containers each containing a sample is provided at the front part of the recess of the casing 2 which is covered by the cover 21. A user of the sample analyzer 1 places in the sample container holder 22 a sample rack that holds multiple sample containers. Measurement is fully automatically performed on the samples which are set in the sample container holder 22 in this manner. A reagent holder 23 is provided at the back of the sample container holder 22. A reagent rack for holding multiple reagent containers each containing a reagent is set in the reagent holder 23. A cuvette holder 24 for holding multiple cuvettes (transparent reaction containers) for use in the measurement is provided at the back of the reagent holder 23. The cuvette holder 24 allows two cuvette racks to be placed therein. Each of the cuvette racks is configured to hold multiple cuvettes. A detector 25 (a measurement section) for performing sample measurement is provided to the right of the cuvette holder 24. A dispenser 26 for dispensing samples and reagents is provided at a higher elevation than the sample container holder 22, the reagent holder 23, the cuvette holder 24, and the detector 25.

The dispenser 26 is movable from front to back and side to side as well as up and down. The dispenser 26 includes a holding part (not shown) for holding a cuvette. A cuvette set in the cuvette holder 24 is held and moved by the holding part. The detector 25 has multiple holes for inserting cuvettes therein. A cuvette held by the dispenser 26 is inserted into one of the holes. The dispenser 26 aspirates a sample (blood plasma) and a reagent, and then dispenses them into the cuvette inserted in the one of the holes of the detector 25. The detector 25 includes a heater. By means of the heater, the sample and reagent dispensed into the cuvette are heated at a constant temperature. A light emitter and a light receiver (not shown) are provided near each hole of the detector 25. The light emitter emits light to a sample to which a reagent has been added. Then, resulting scattered light or transmitted light is received by the light receiver. The cover 21 is a light-shielding cover. Closing the cover 21 prevents the detector 25 from being affected by ambient light during the measurement. The controller 6 analyzes data of the received light, and thereby obtains analysis results regarding blood coagulation.

Specifically, PT (prothrombin time), APTT (activated partial thromboplastin time), Fbg (fibrinogen), and the like are measured by a coagulation time method with which a coagulation time is determined by observing the coagulation process of a sample based on a change in the amount of the received scattered light. Moreover, AT3 (antithrombin III) and the like are measured by a synthetic substrate method with which a change amount in absorbance during a period of one minute is determined by observing the light transmitted through a sample to which a reagent for the synthetic substrate method has been added. Furthermore, D-Dimer and the like are measured by immunonephelometry with which a change amount in absorbance during a period of one minute is determined by observing the light transmitted through a sample to which a reagent for a latex agglutination method has been added.

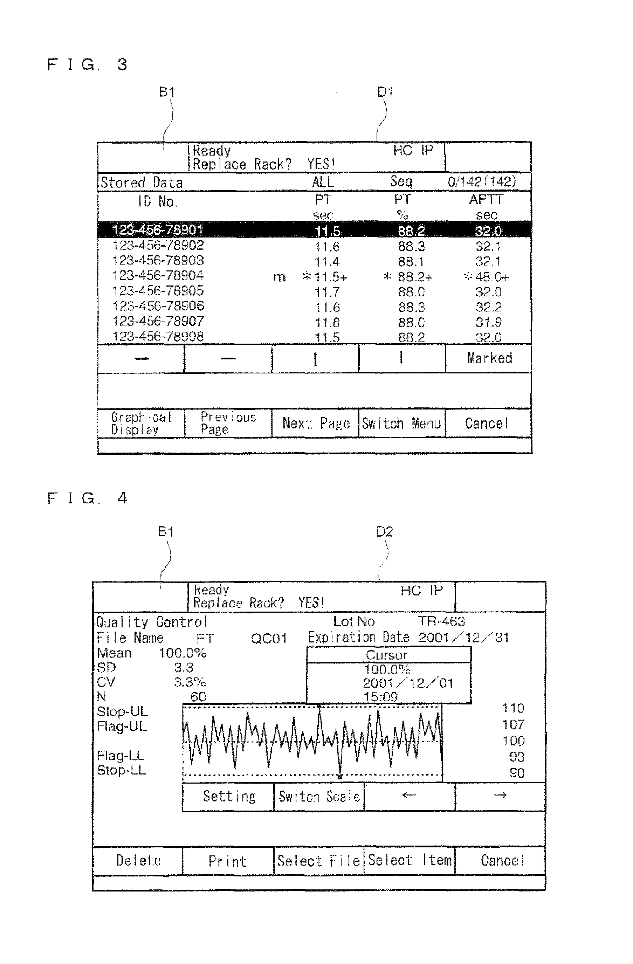

The user can set, through operating the sample analyzer 1, any one or more measurement items (PT, APTT, Fbg, AT3, D-Dimer, etc.) for the sample analyzer 1. The sample analyzer 1 is configured to measure a sample for the measurement items that have been set, and display analysis results on the display 3. FIG. 3 shows an example of an analysis result screen. As shown in FIG. 3, an analysis result screen D1 displays a list that shows sample numbers of measured samples and shows measurement data (numerical data) that has been obtained for the respective measurement items for each measured sample. The sample numbers are those read by a bar code reader (not shown) from bar code labels affixed to respective sample containers of the measured samples, or those specified by the user operating the touch panel 4. A button B1 for calling up a maintenance menu screen, which will be described below, is provided at the top left of the analysis result screen D1.

The sample analyzer 1 is configured to perform quality control by measuring a quality control sample such as a control plasma or pooled plasma (e.g., "control plasma N for blood coagulation test" available from Sysmex Corporation). Through the quality control, the user can monitor whether the sample analyzer 1 is precisely performing the sample measurement. In order to perform quality control measurement, the user sets quality control samples in the sample container holder 22, inputs quality control sample numbers (sample numbers which are used only when the quality control is performed) and measurement items, and then instructs the sample analyzer 1 to start the quality control measurement. After the quality control measurement is performed, the results of the quality control measurement are displayed on the display 3. FIG. 4 shows an example of a quality control screen. As shown in FIG. 4, a quality control screen D2 displays a chart which shows a history of the results of the quality control measurement, the lot number of the quality control samples, the standard deviation of data obtained from the quality control measurement, the reproducibility of the data obtained from the quality control measurement, and the like. Similar to the analysis result screen D1, the button B1 for calling up the maintenance menu screen, which will be described below, is provided at the top left of the quality control screen D2.

The sample analyzer 1 is configured to transmit quality control measurement data, which is obtained in the above manner, to an external server (i.e., a mail server 101 described below). This makes it possible to perform online quality control for the same type of sample analyzers installed in respective different facilities (i.e., external quality control). Each time a quality control sample is measured, the quality control measurement data is transmitted to the mail server 101. Further, the controller 6 of the sample analyzer 1 is configured to transmit status information about the sample analyzer 1 to the mail server 101, which status information contains an error history, the number of operations performed by consumable components (the light emitter, a syringe attached to the dispenser 26, etc.), version information about a control program, and setting values of various parameters. The transmission of the status information is performed, for example, when the sample analyzer 1 is powered on and initialized, or at a predetermined time every day.

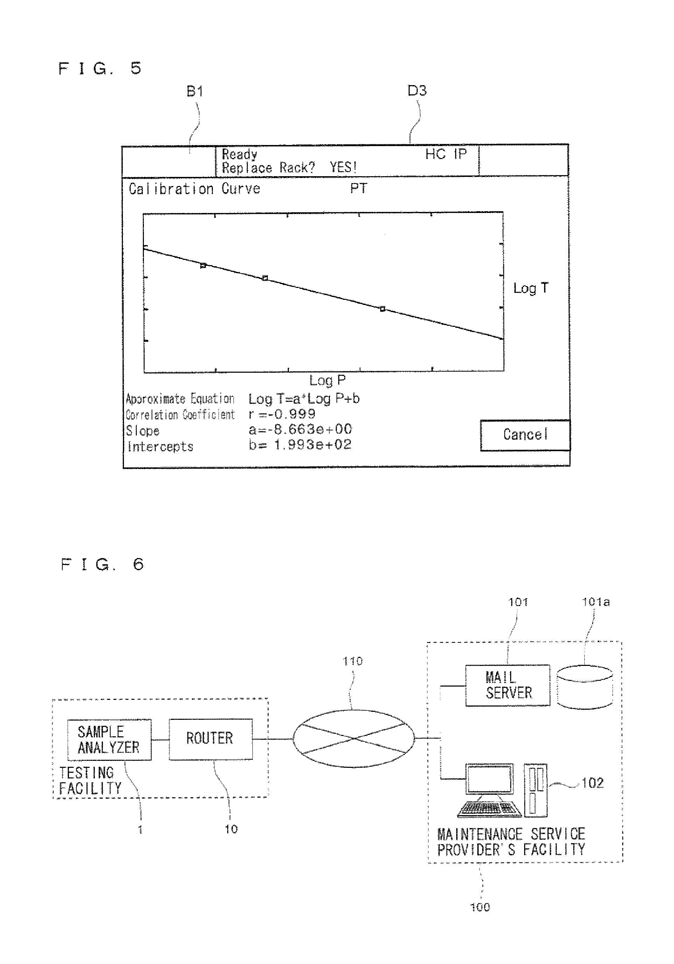

The sample analyzer 1 is configured to compare a coagulation time or the like obtained from measurement results with a calibration curve stored in the controller 6, thereby obtaining analysis results for the measurement items. The sample analyzer 1 creates the calibration curve by measuring a calibrator (i.e., a standard sample). The calibration curve is created in the following manner: the user first sets the calibrator in the sample container holder 22; causes the sample analyzer 1 to display calibration curve data of a target measurement item; inputs, for example, the assigned value of the calibrator; and instructs the sample analyzer 1 to start measurement. The sample analyzer 1 is configured to display on the display 3 the calibration curve created in this manner. FIG. 5 shows an example of a calibration curve screen. As shown in FIG. 5, a calibration curve screen D3 displays a measurement item, a graph showing a calibration curve, an approximate equation representing the calibration curve, the correlation coefficient, slope, and intercepts of the calibration curve, etc. Similar to the analysis result screen D1 and the quality control screen D2, the button B1 for calling up the maintenance menu screen, which will be described below, is provided at the top left of the calibration curve screen D3.

The sample analyzer 1 according to the present embodiment as described above is installed in a hospital or a testing facility such as a laboratory. The sample analyzer 1 is connected via a communication network (the Internet) to a maintenance service system which is installed in a facility of a service provider providing a maintenance service and academic advice, in such a manner as to allow the sample analyzer 1 to perform data communication with the maintenance service system. This makes it possible to receive a maintenance service and academic advice from the service provider.

FIG. 6 is a schematic diagram briefly showing the maintenance service system. As shown in FIG. 6, a maintenance service system 100 includes the mail server 101 and a client computer 102 which is used by a service person and an academic specialist. The mail server 101 has SMTP server functions and POP server functions. That is, the mail server 101 uses SMTP (Simple Mail Transfer Protocol) to receive an email transmitted from the sample analyzer 1, and stores the email in a mailbox (i.e., an area in a hard disk) of the mail server 101. The mail server 101 uses POP (Post Office Protocol) to transmit emails in the mailbox to the client computer 102 in response to a request from the client computer 102.

Further, the mail server 101 has web server functions. The mail server 101 has a facility information database 101a which stores information about facilities which are users of sample analyzers (facility information). The mail server 101 is configured to convert the facility information stored in the facility information database 101a into data in HTML format, and transmit the data to the client computer 102 (or to a different computer that is connected to a communication network 110). This allows the service person using the client computer 102 to browse the facility information by means of a web browser program.

Moreover, the mail server 101 is configured to receive measurement data of quality control samples from multiple testing facilities, create statistical data based on the measurement data, and provide the statistical data to the respective facilities through the Web (i.e., external quality control). Furthermore, the mail server 101 is configured to receive the status information from the sample analyzer of each testing facility, cumulatively store past status information about each sample analyzer, and manage the status information. The service person uses the status information when providing a maintenance service.

The sample analyzer 1 is connected to a router 10 which is installed in the testing facility where the sample analyzer 1 is installed. The sample analyzer 1 is configured to transmit/receive data to/from the communication network 110 via the router 10. The router 10 has DNS (Domain Name System) server functions and DHCP (Dynamic Host Configuration Protocol) server functions. The sample analyzer 1 requests the router 10 to assign an IP address, a subnet mask, and the like to the sample analyzer 1. The sample analyzer 1 sets the IP address and the subnet mask, which the router 10 has assigned to the sample analyzer 1, as its own IP address and subnet mask (i.e., as the IP address and subnet mask of the controller 6). The sample analyzer 1 requests, at the time of transmitting data to a particular host, the router 10 for an IP address that is associated with the name of the host, and transmits the data to the IP address which the sample analyzer 1 is informed of from the router 10.

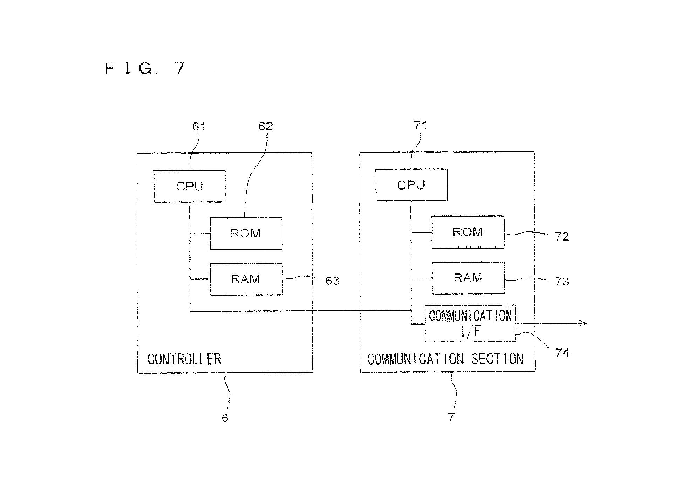

FIG. 7 is a block diagram showing a part of the configuration of the sample analyzer 1 according to the present embodiment. As shown in FIG. 7, the controller 6 includes a CPU 61, a ROM 62, and a RAM 63. The CPU 61, the ROM 62, and the RAM 63 are connected with one another via a bus in such a manner as to allow them to perform data communication with one another. The communication section 7 includes a CPU 71, a ROM 72, a RAM 73, and a communication interface 74. The CPU 71, the ROM 72, the RAM 73, and the communication interface 74 are connected with one another via a bus in such a manner as to allow them to perform data communication with one another. The CPU 61 of the controller 6 is connected to the CPU 71 via a bus.

The ROM 62 stores a control program for controlling the respective components of the sample analyzer 1. The sample analyzer 1 is configured to perform, through the execution of the control program by the CPU 61, operations such as the above-described sample analysis, quality control, calibration curve creation, etc. The ROM 62 is a rewritable flash memory, in which various setting data are written. Examples of the setting data include the email address of the service person which will be described below, the email address of the sample analyzer 1 (an email address is set for each of the sample analyzers of the respective testing facilities), a link address linked to a web page that contains contact information about the facility where the sample analyzer 1 is installed, and the model name of the sample analyzer 1. The email address of the sample analyzer 1 contains an analyzer ID assigned to the sample analyzer 1. Such an analyzer ID is assigned to each of the sample analyzers of the respective testing facilities.

As described below, the communication section 7 is configured to transmit an email to the service person. The email transmission is performed when the CPU 71 executes a communication program stored in the ROM 72. The communication interface 74 is an Ethernet (registered-trademark) controller. The communication section 7 is configured to transmit/receive data to/from the outside via the communication interface 74.

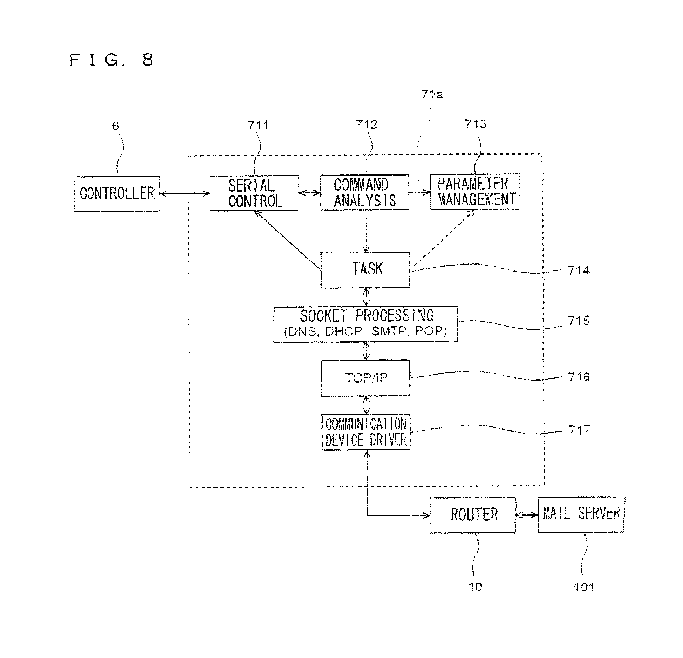

FIG. 8 is a block diagram showing functions of the communication program. As shown in FIG. 8, a communication program 71a includes the following function blocks: a serial control block 711, a command analysis block 712, a parameter management block 713, a task block 714, a socket processing block 715, a TCP/IP block 716, and a communication device driver block 717. The serial control block 711 controls serial communication which is performed with the controller 6, thereby performing data transmission/reception with the controller 6. The command analysis block 712 analyzes a command received from the controller 6 and generates response data to respond to the controller 6. Based on the received command, the command analysis block 712 generates an event to the task block 714. The parameter management block 713 manages a parameter received from the controller 6. The task block 714 manages the status of the communication program 71a (the status of each task being performed by the CPU 71). The socket processing block 715 has DHCP client functions, DNS client functions, SMTP client functions, and POP client functions. The socket processing block 715 performs the DHCP client functions, DNS client functions, SMTP client functions, and the POP client functions in accordance with instructions from the task block 714. Each of these functions, when performed, generates a processing result event to the task block 714. The TCP/IP block 716 divides transmission data provided from the socket processing block 715, thereby generating IP packets, and sends the IP packets to the communication device driver block 717. The TCP/IP block 716 re-constructs data from IP packets received from the communication device driver block 717, and sends the data to the socket processing block 715. The communication device driver block 717 drives the communication interface 74.

The communication section 7 is configured such that the functions thereof are specifically focused on transmitting outgoing emails (to a specific destination). That is, the communication section 7 has the DNS client functions, DHCP client functions, SMTP client functions, and the POP client functions (used for POP before SMTP), which are necessary functions for email transmissions via the Internet. The communication section 7 does not have functions for receiving data that is transmitted from the client computer 102 for the purpose of providing a maintenance service (e.g., an email that contains an answer to a question from the user of the sample analyzer 1, data requesting to start a video conference, and command data for remote control).

### [Operations of Sample Analyzer]

Next, operations of the sample analyzer 1 configured as described above are described. The user places sample containers, each of which contains a sample, in a sample rack, and sets the sample rack in the sample container holder 22. Then, the user performs necessary settings, for example, inputs sample numbers and measurement items. Thereafter, the user touches with a finger a measurement start button (not shown) displayed on the display 3. The touch panel 4 detects an instruction that corresponds to the position touched by the user. Accordingly, the sample analyzer 1 is instructed to start operating. In this manner, the sample analyzer 1 starts measuring the samples. When the measurement has been completed, the analysis result screen is displayed. As described above, after quality control measurement has been performed, the quality control screen is displayed, and after a calibration curve is created, the calibration curve screen is displayed. In each of such displayed screens including the analysis result screen, the quality control screen, and the calibration curve screen, the button B1 (see FIGS. 3 to 5) for calling up the maintenance menu screen is shown at the top left of the displayed screen. If the user wishes to use a maintenance service or academic advice, for example, if the user cannot fully comprehend information shown in the displayed screen since the user has difficulty comprehending some of the information, or if the user does not know how to handle an abnormality that has occurred in the sample analyzer 1, or if the user wishes to know information that is obtained from sample analysis results through academic estimation, then the user can request, in such a manner as described below, a maintenance service or academic advice from the service provider.

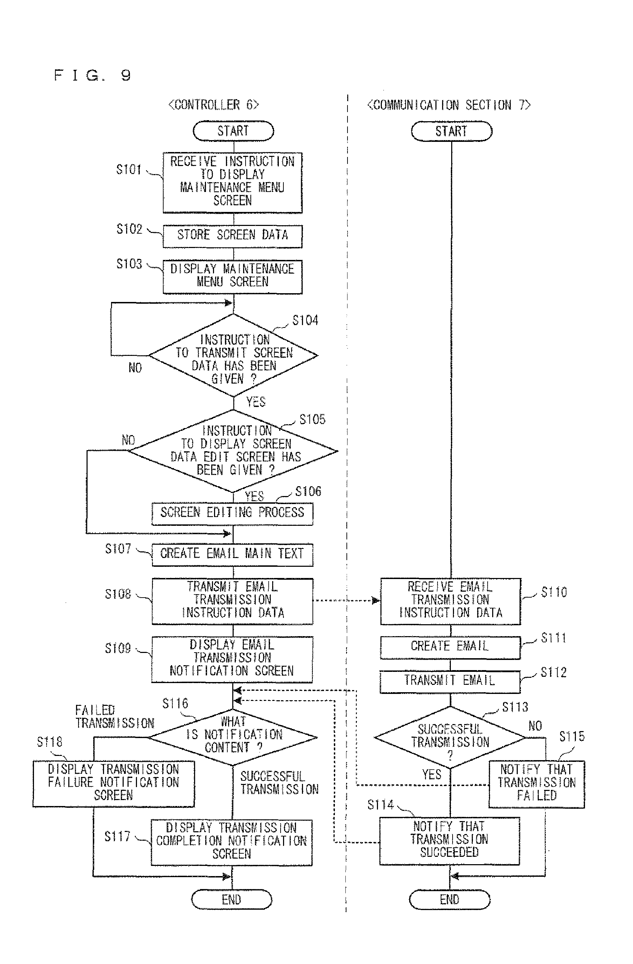

FIG. 9 is a flowchart showing a sequence of processing which the controller 6 and the communication section 7 of the sample analyzer 1 according to the present embodiment perform when the sample analyzer 1 performs a maintenance service requesting operation. The user touches with a finger the button B1 shown in a displayed screen with which the user is having a problem, thereby instructing the sample analyzer 1 to display the maintenance menu screen. When an event of receiving the instruction to display the maintenance menu screen has occurred (step S101), the CPU 61 of the controller 6 performs processing as described below. First, the CPU 61 captures the currently displayed screen, that is, the screen though which the instruction to display the maintenance menu screen has been given, and stores the screen in the RAM 63 in the form of image data (hereinafter, screen data)(step S102). Then, the CPU 61 switches the currently displayed screen to the maintenance menu screen, and causes the display 3 to display the maintenance menu screen (step S103).

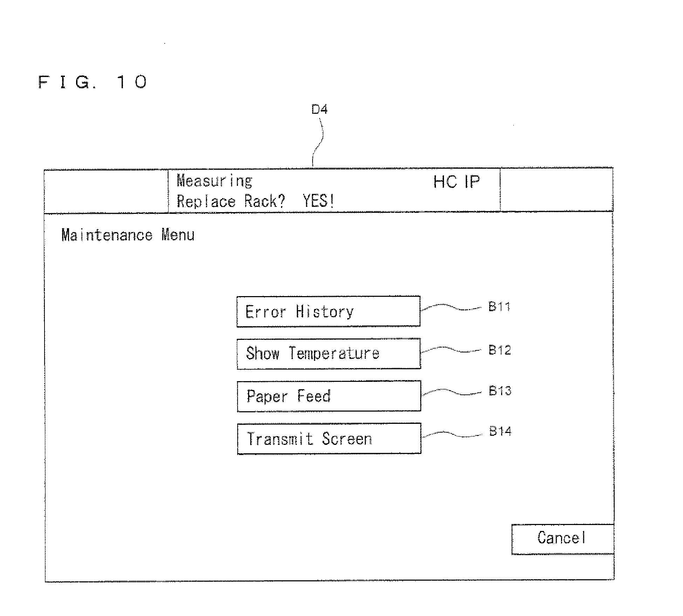

FIG. 10 shows the maintenance menu screen. As shown in FIG. 10, a maintenance menu screen D4 includes a button B11 for displaying an error history of the sample analyzer 1, a button B12 for displaying internal temperatures of the sample analyzer 1, a button B13 for the paper feed of the printer 5, and a button B14 for transmitting the captured screen data to the maintenance service provider. When the position of one of the buttons B11 to B14 is touched by a finger, the CPU 61 determines that one of the buttons B11 to B14, which corresponds to the touched position, has been selected.

The CPU 61 determines whether the button B14 has been selected, that is, whether an instruction to transmit the captured screen data has been received (step S104). Here, the CPU 61 also determines whether any of the other buttons B11 to B13 has been selected. If any of them has been selected, a process corresponding thereto is performed. Thus, for example, if the button B11 is selected, a history of errors that occurred in the past in the sample analyzer 1 is displayed in the form of a list. Similarly, if the button B12 is selected, internal temperatures of the sample analyzer 1, for example, the temperature of the detector 25 which is heated at the constant temperature by the heater, and the temperature of the reagent holder 23 which includes a cooling device for cooling reagents, are displayed. Further, if the button B13 is selected, feeding of a printing paper is performed by the printer 5. At step S104, if no button is selected (NO at step S104), the CPU 61 repeats the process at step S104, waiting for an input. On the other hand, if the button B14 is selected (YES at step S104), the CPU 61 displays a screen for confirming whether to edit the screen data stored in the RAM 63, waiting for an input from the user (step S105). The screen includes a first button for instructing to display a screen data edit screen and a second button for instructing to transmit the screen data without editing the screen data. The user selects the first button in the case of editing the screen data, and selects the second button in the case of instructing to transmit the screen data without editing the screen data.

If an instruction to display a screen data edit screen has been received from the user at step S105, that is, if the selection of the first button has been detected (YES at step S105), the CPU 61 displays the screen data edit screen (not shown), and performs a screen editing process (step S106). The edit screen shows drawing tools for drawing characters, figures, or a combination of characters and figures to edit the screen data stored in the RAM 63. The user uses the drawing tools to draw characters, figures, or a combination of characters and figures on the edit screen, thereby specifying information on the screen for which the user wishes to receive an explanation, or writing a question on the screen. In this manner, the user can edit the screen data. The edit screen includes a button for instructing to end the editing of the screen data. When the user performs an operation of selecting the button, the screen data stored in the RAM 63 is overwritten with the edited screen data. Thereafter, the processing proceeds to step S107.

If no instruction to display a screen data edit screen, but instead an instruction to transmit the screen data, is received from the user at step S105, that is, if the selection of the second button is detected (NO at step S105), the CPU 61 advances the processing to step S107.

At step S107, the CPU 61 generates data for creating an email that contains the screen data stored in the RAM 63 as an attachment (step S107).

Hereinafter, the process at step S107 is described in detail. In this process, the main text of the email is created. The screen data stored in the RAM 63 is attached to the main text of the email, and the aforementioned link address stored in the ROM 62 and an error history about errors that occurred after the previous system shutdown are written in the main text. It should be noted that each time an error occurs, the CPU 61 of the controller 6 cumulatively stores the error in the RAM 63 as the error history.

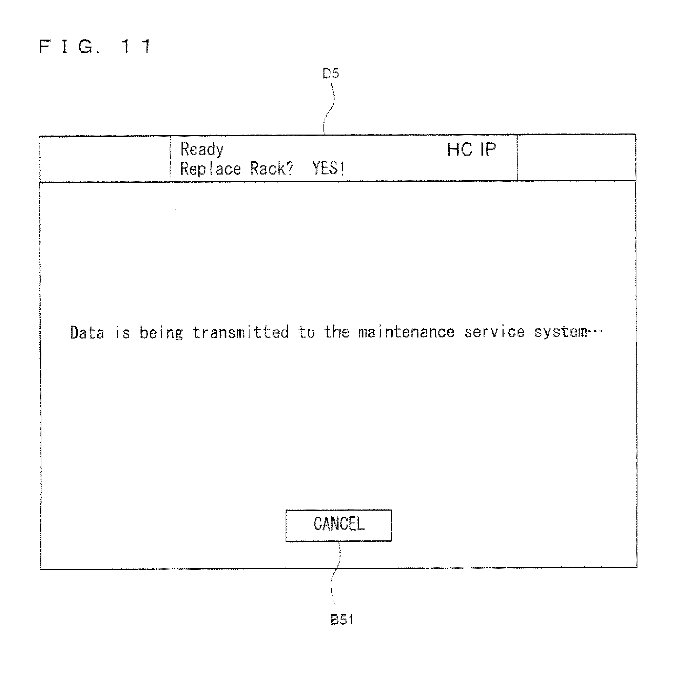

Next, the CPU 61 transmits, to the communication section 7, email transmission instruction data which contains data of the email main text created in the above manner and the email address of the service person which is stored in the ROM 62 (step S108), and causes the display 3 to display a screen indicating that an email is currently being transmitted (an email transmission notification screen)(step S109). FIG. 11 shows the email transmission notification screen. As shown in FIG. 11, an email transmission notification screen D5 displays the following message: "Data is being transmitted to the maintenance service system... ". The email transmission notification screen D5 includes a cancel button B51. When the user's operation of touching with a finger the cancel button is detected, the email transmission to the maintenance service system is interrupted.

The communication section 7 receives the email transmission instruction data (step S110), and the CPU 71 creates an email (step S111). Hereinafter, the process at step S111 is described in detail. The CPU 71 sets, as the destination of the email, the email address of the service person which is contained in the received email transmission instruction data, and reads the model name of the sample analyzer 1 from the ROM 62 to generate an email header which contains the following predetermined character string as a subject: "DB-700 Screen Shot". The email header contains, as a sender's address (i.e., source information), the email address of the sample analyzer 1, which is stored in the ROM 62. The CPU 71 creates an email by using the email header generated in the above manner as well as the main text and the screen data which are contained in the received email transmission instruction data, such that the email contains the screen data as an attachment.

The CPU 71 drives the communication interface 74 to transmit the created email to the mail server 101 (step S112). In the present embodiment, according to the SMTP server functions of the mail server 101, authentication is performed by POP before SMTP. The email is received when the authentication is successful. Next, the CPU 71 determines whether the email transmission has succeeded (step S113). If the email transmission has succeeded (YES at step S113), the CPU 71 notifies the controller 6 of the successful email transmission (step S114). If the email transmission has failed (NO at step S113) for the reason that, for example, a LAN cable is not connected, an IP address is not set, the router 10 is not operating, a DNS server is not responding, or a connection cannot be established with the POP3 port or SMPT port of the mail server 101, then the CPU 71 notifies the controller 6 of the failed email transmission (step S115).

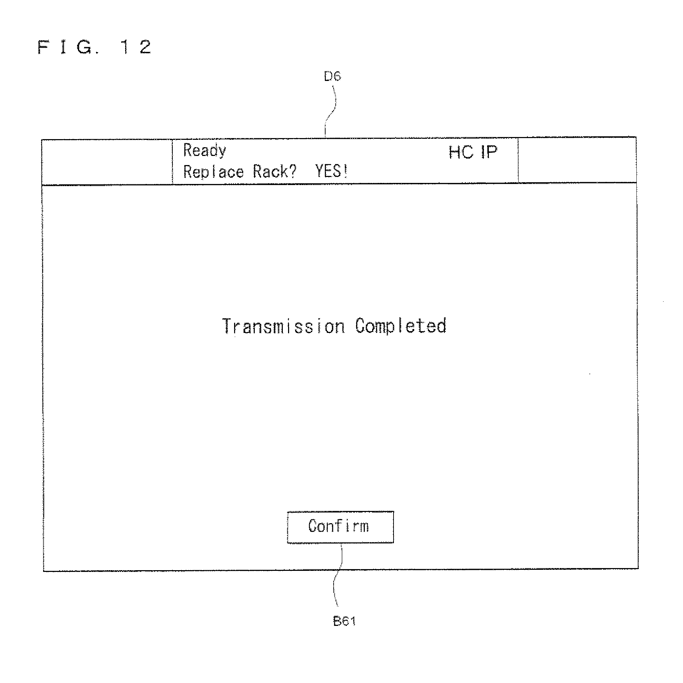

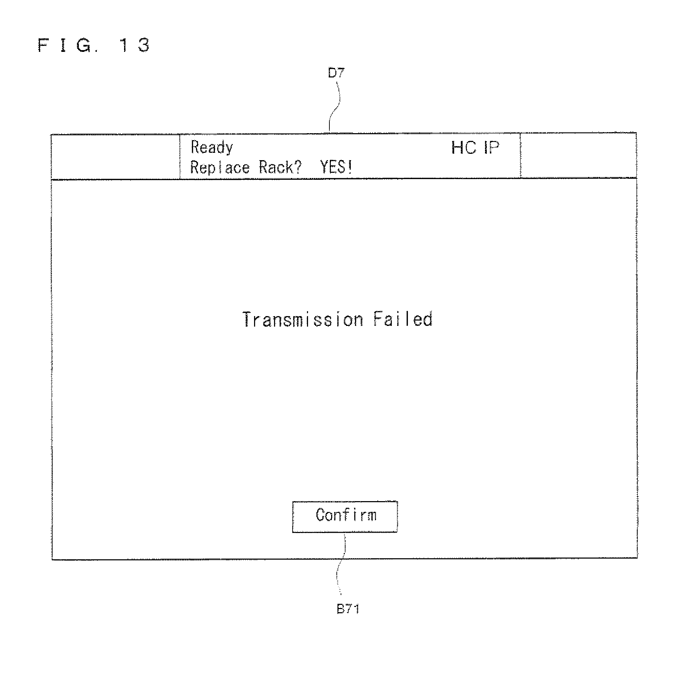

The controller 6 receives notification data from the communication section 7, which notification data indicates whether the email transmission has succeeded or failed (step S116). If the received notification data indicates that the email transmission has succeeded ("SUCCESSFUL TRANSMISSION" at step S116), then the CPU 61 causes the display 3 to display a transmission completion notification screen (step S117). On the other hand, if the received notification data indicates that the email transmission has failed ("FAILED TRANSMISSION" at step S116), then the CPU 61 causes the display 3 to display a transmission failure notification screen (step S118). FIG. 12 shows the transmission completion notification screen, and FIG. 13 shows the transmission failure notification screen. As shown in FIG. 12, a transmission completion notification screen D6 displays a message "Transmission Completed". As shown in FIG. 13, a transmission failure notification screen D7 displays a message "Transmission Failed". The transmission completion notification screen D6 includes a confirmation button B61 and the transmission failure notification screen D7 includes a confirmation button B71. When the user's operation of touching with a finger the confirmation button B61 or B71 is detected, the displayed screen is switched to the maintenance menu screen D4, and the maintenance service requesting operation ends. It should be noted that if, for example, the email transmission has failed, the user can select the button B14 in the maintenance menu screen D4 again to give an instruction to transmit the same email.

If the user wishes to newly transmit an email for a screen that is different from a screen for which the user has already transmitted an email, the user causes the display 3 to display the screen for which the user wishes to newly transmit an email. Then, the user selects the button B1 shown at the top left of the screen to switch the displayed screen to the maintenance menu screen. At the time when the displayed screen is switched, the screen displayed prior to the maintenance menu screen is stored in the RAM 63 as a screen for which an email is to be transmitted. At this point, screen data of the screen (i.e., new screen data) is written over the previously stored screen data in the RAM 63. In other words, in the RAM 63, a storage area for storing screen data to be transmitted has a capacity for storing screen data of only one screen. Therefore, each time the button B1 for calling up the maintenance menu screen is selected, the screen data in the storage area is overwritten with new screen data. Since the RAM 63 is required to include the storage area having a capacity for storing screen data of only one screen, the capacity of the RAM 63 can be reduced. This makes it possible to reduce the production cost. Thereafter, the user selects the button B14 in the maintenance menu screen D4 again to give a screen data transmission instruction to the sample analyzer 1. Accordingly, an email that includes, as an attachment, the new screen data stored in the RAM 63 is transmitted.

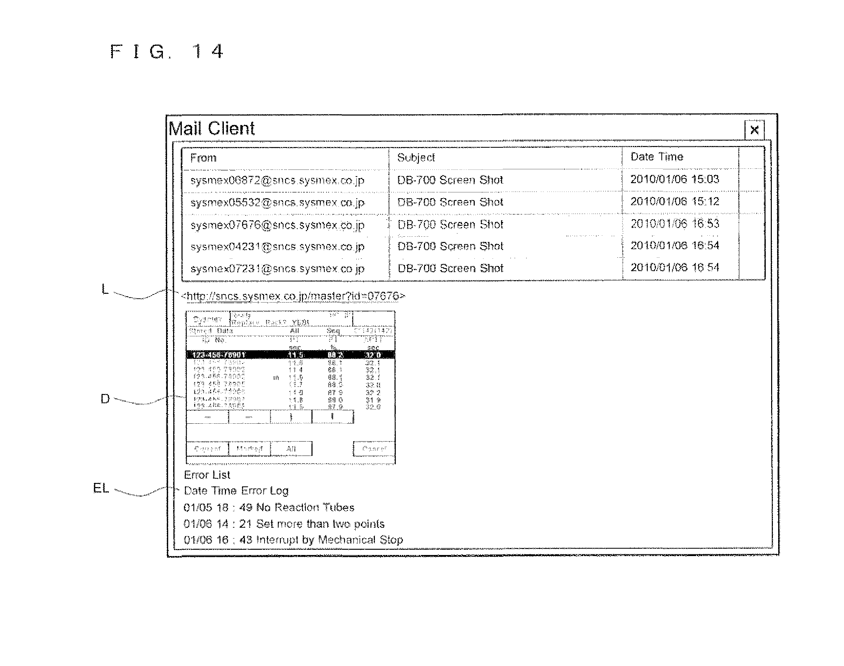

After the email is transmitted in the above manner, the email is stored in the mailbox of the mail server 101. When the service person starts a mailer of the client computer 102 and email retrieval from the mail server 101 by POP is performed, the email stored in the mailbox is transmitted to the client computer 102 and the contents of the email are displayed on a display of the client computer 102. FIG. 14 shows an example of an email, for requesting a maintenance service or academic advice, which is displayed on the display of the client computer 102. As shown in FIG. 14, senders' email addresses of emails transmitted from respective sample analyzers are different among the sample analyzers. Each sender's email address contains an analyzer ID for identifying a sample analyzer that corresponds to the sender's email address. In the example of FIG. 14, the email address of a sample analyzer of which the analyzer ID is "07676" is "sysmex07676@sncs.sysmex.co.jp". This allows the service person to identify a sample analyzer that is the source of an email, by simply referring to the sender's email address of the email. In this example, the subject of an email transmitted from the sample analyzer 1 is "DB-700 Screen Shot". DB-700 represents the model name of the sample analyzer 1. This allows the service person to identify the model of the sample analyzer 1 which is the source of the email, by simply referring to the subject of the email. Since the displayed respective senders' information contain analyzer IDs and the displayed respective subjects contain model names, the service person can identify, without opening the emails, the analyzers that are the sources of the respective emails.

The main text of each email contains a link L which is a link to facility information about a testing facility where a sample analyzer that is the source of the email is installed, and also contains an error history EL. The link L (which is a link address) is set for each sample analyzer, and is linked to facility information about a testing facility where the sample analyzer is installed. The error history EL contains dates and times when errors occurred as well as the details of the errors. As described above, screen data is attached to an email transmitted from the sample analyzer 1. In the example shown in FIG. 14, a screen D, which is an attachment to the email, is displayed in an area where the main text of the email is displayed. The example of FIG. 14 shows an email that is transmitted in a case where the user has not performed screen editing.

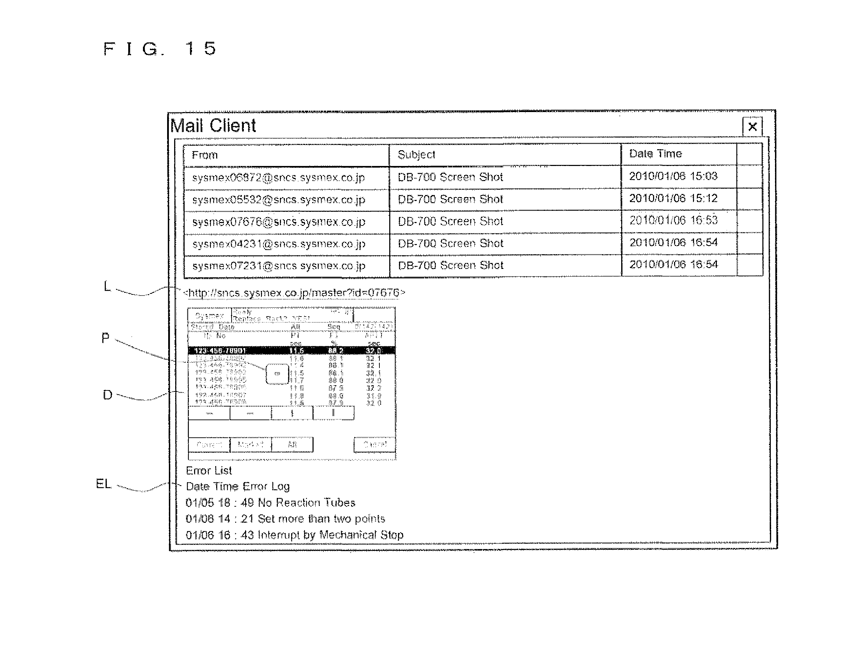

FIG. 15 shows another example of an email, for requesting a maintenance service or academic advice, which is displayed on the display of the client computer 102. FIG. 15 shows an email that is transmitted in a case where the user has performed screen editing. As shown in FIG. 15, a screen D, which is based on screen data attached to the email, includes a figure P drawn by the user. By referring to the screen D, the service person can easily recognize that the user is wishing to have an explanation of the information indicated by the figure P.

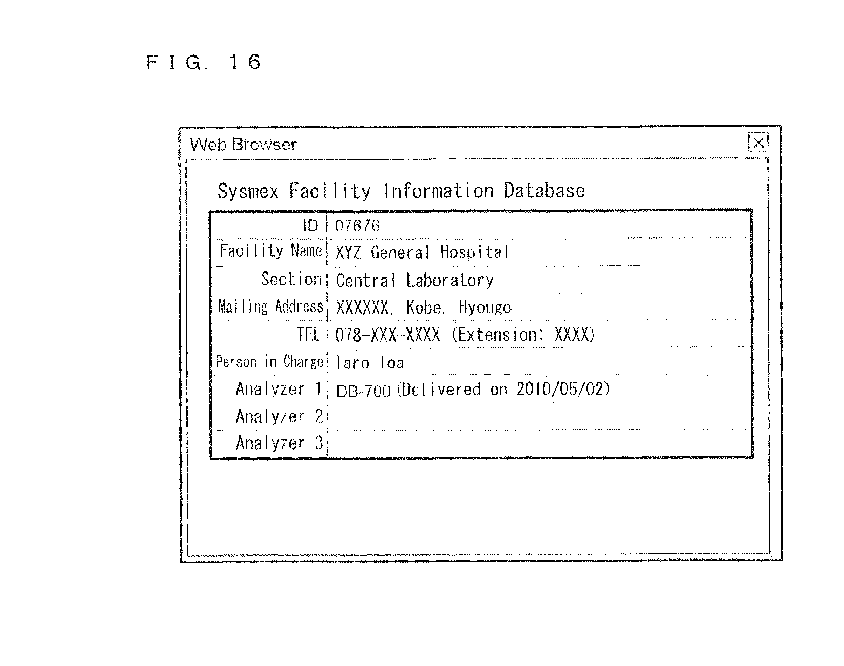

By referring to such a screen D attached to an email, the service person can easily know what screen the user is wishing to receive a maintenance service or academic advice about. Also, by referring to the error history EL, the service person can easily know the status of the sample analyzer 1. If the service person wishes to know detailed information about the testing facility where the sample analyzer 1 is installed, such as the telephone number, mailing address, or facility name, for the reason that, for example, it is necessary to contact the testing facility by telephone or the like or to visit the testing facility, or the service person has forgotten the facility ID of the testing facility, then the service person operates input means, such as a mouse, of the client computer 102 to point to the link L. Accordingly, a web browser of the client computer 102 starts, and a request for HTML data that contains facility information about the testing facility is transmitted to the mail server 101. In response to the request, the mail server 101 reads the facility information about the testing facility from the facility information database 101 a, and creates and transmits HTML data to the client computer 102. Upon receiving the HTML data, the client computer 102 displays the facility information in a web browser screen. FIG. 16 shows an example of the facility information displayed by the client computer 102. As shown in FIG. 16, the display of the client computer 102 displays the facility information which contains the facility ID, the facility name, the name of a section to which the service person provides a service, the mailing address of the facility, the telephone number of the facility, the name of a person in charge of a sample analyzer installed in the facility, the model name of the sample analyzer, etc. This allows the service person to easily browse the facility information. By using the facility information, the service person can contact the testing facility which is the source of the email, and provide a maintenance service. For example, the service person may call the telephone number contained in the facility information, and explain over the phone (i.e., verbally) about the screen attached to the email.

As described above, the sample analyzer 1 receives by means of the controller 6 an instruction to transmit a screen. The sample analyzer 1 then transmits, to the mail server 101 by means of the communication section 7, screen data (a screen D) which indicates the screen for which the transmission instruction has been received, together with the analyzer ID and the link L associated with the sample analyzer 1. This allows the service person to promptly identify the source of the email. This makes it possible for the user to promptly receive a maintenance service or academic advice. The communication section 7 of the sample analyzer 1 has a function of transmitting an email for requesting a maintenance service or academic advice, but does not have a function of receiving an email (e.g., an email containing an answer to a question from the user of the sample analyzer 1). This lowers the production cost of the sample analyzer 1, and yet allows the user to promptly receive a maintenance service or academic advice. The screen data of a screen, which the user has instructed to transmit, is attached to the email. Accordingly, when the user receives the maintenance service from the service person or the like, for example, over the phone, both of the user and the service person can view the screen. This allows the user to easily tell the service person over the phone (i.e., verbally) what part of the information in the screen the user is having a problem with, and allows the service person to explain to the user over the phone (i.e., verbally) how to handle the user's problem (i.e., what operation to perform) on the screen. Since the service person or the like can view the screen, the service person can easily know what maintenance service or academic advice the user wishes to receive regarding the screen.

In each screen displayed by the sample analyzer 1, when the user performs an operation of pointing to the button positioned at the top left of the screen for calling up the maintenance menu (hereinafter, a maintenance menu call up button), screen data of the screen through which the operation has been received is stored as screen data to be transmitted. Therefore, other than the maintenance menu call up button, it is not necessary to provide a button, icon, menu or the like for instructing to store the screen data. Thus, the display area can be used efficiently. The sample analyzer as described in the above embodiment is structured such that all the functions of the sample analyzer are accommodated in a single casing. Therefore, the display of the sample analyzer is designed to be small for the purpose of reducing the size and the production cost of the sample analyzer. It is desired for such a sample analyzer with a small display to use the limited display area efficiently. Accordingly, configuring each screen in the manner described above is particularly advantageous for such a small-sized sample analyzer.

The maintenance menu call up button is displayed at a predetermined position on the display 3 regardless of the type of a screen displayed on the display 3, for example, regardless of whether the screen displayed on the display 3 is the analysis result screen D1, the quality control screen D2, or the calibration curve screen D3. This allows the user to easily memorize the position of the maintenance menu call up button.

The sample analyzer 1 is configured to transmit data for requesting a maintenance service in the form of an email to which screen data is attached. For this reason, a computer having general-purpose email client software installed therein will suffice to serve as the client computer used by the service person or the like. Thus, it is not necessary for the maintenance service provider to own an apparatus that is dedicated for receiving screen data. This allows the maintenance service provider to easily, and at low cost, construct the maintenance service system for receiving screen data as described above from the sample analyzer 1.

### (Other Embodiments)

The above embodiment describes that characters, figures, or a combination of characters and figures is drawn in a screen (a screen stored in the RAM 63) through which an instruction to display the maintenance menu screen has been received, and that the screen data of the screen on which such editing has been made is transmitted. However, the present invention is not limited thereto. The present invention need not have such screen data editing function. The screen through which an instruction to display the maintenance menu screen has been received may be attached to an email to be transmitted, without any editing made on the screen.

In the above-described embodiment, data for requesting a maintenance service is in the form of an email to which screen data is attached. However, the present invention is not limited thereto. Data for requesting a maintenance service, which contains screen data, may be in any original format. In such a case, the client computer is required to have functions of interpreting the data in the original format and displaying the screen data.

The above embodiment describes that the sample analyzer 1 is configured to transmit an email to which screen data of a single screen is attached. However, the present invention is not limited thereto. The sample analyzer 1 may be configured to receive a screen capturing instruction for multiple screens, store screen data of the multiple screens, and transmit an email to which the screen data of the multiple screens is attached.

The above embodiment describes that when the button B1 for calling up the maintenance menu screen D4 is selected, the screen displayed at the time is captured, and when the button B14 for instructing to transmit the screen is selected in the maintenance menu screen D4, an email to which the screen data of the captured screen is attached is transmitted. However, the present invention is not limited thereto. As an alternative, not software keys displayed on the display 3 but push button switches may be provided on the casing 2 as hardware components. When the push button switches are operated, the screen displayed at the time may be captured, and an email to which the captured screen is attached may be transmitted.

The above embodiment describes that an email that contains an error history in its main text is transmitted. However, the present invention is not limited thereto. An email that contains, as a main text or an attachment, not only an error history but also status information about the status of the sample analyzer 1 may be created and transmitted. Here, the status information may contain the number of operations performed by consumable components (the light emitter, a syringe attached to the dispenser 26, etc.), version information about the control program, and setting values of various parameters, and measurement data of quality control samples.

The above embodiment describes that the sample analyzer 1 transmits an email via the router 10 to the mail server 101 which has SMPT server functions and POP server functions, and the mail server 101 stores the email in its mailbox. However, the present invention is not limited thereto. The sample analyzer 1 may transmit an email to an SMPT server which is provided separately from the mail server 101, and the SMPT server may transmit the email to the mail server 101. Then, the mail server 101 may store the email in its mailbox. Further, the maintenance service system 100 need not include the mail server 101. The sample analyzer 1 may transmit an email to an SMPT server which is provided outside the maintenance service system 100, and then the email may be stored in a mailbox within a POP server which is provided outside the maintenance service system 100. In this case, the client computer 102 used by the service person is required to access the POP server to receive the email. Further alternatively, an IMAP server or a webmail server may be used instead of a POP server. In this case, the client computer 102 refers to an email stored in the mail server, keeping the email left in the mail server.

In the above-described embodiment, the router 10 has DNS server functions and DHCP server functions. However, the router 10 need not have these server functions. A DHCP server may be provided separately from the router 10 so as to be located downstream from the router 10 in a LAN (the LAN includes the sample analyzer 1 and the router 10). Also, a DNS server may be provided separately from the router 10 in the LAN or in a communication network (the internet).

The above embodiment describes that the controller 6 includes the CPU 61 and the communication section 7 includes the CPU 71. The CPU 61 stores screen data in the RAM 63 and creates an email main text, and the CPU 71 creates and transmits an email containing the main text. However, the present invention is not limited thereto. As an alternative, the CPU 61 of the controller 6 may store screen data in the RAM 63 and create an email in addition to creating the main text of the email, and the CPU 71 of the communication section 7 may transmit the email. As a further alternative, the communication section 7 need not include a CPU, and the CPU 61 of the controller 6 may perform all of the following: store screen data in the RAM 63, create an email including creating the main text of the email, and drive the communication interface 74 to transmit the email.

The controller 6 or the communication section 7 may set, on screen data which is an attachment, a password for opening the attachment. This makes it possible to safely transmit a screen that contains personal information about a subject.

In the above-described embodiment, the sender's email address of a transmitted email contains an analyzer ID. However, as an alternative, the sender's email address may contain a facility ID for identifying a facility where a sample analyzer that is the source of the email is installed, As a further alternative, the sender's email address may contain the name of the facility. This makes it possible to more easily identify the facility where the sample analyzer that is the source of the email is installed.

In the above-described embodiment, an analyzer ID is contained in the sender's email address of an email. However, as an alternative, an analyzer ID may be contained in the subject of the email or written in the main text of the email.

The above embodiment describes that the CPU 71 of the communication section 7 is configured to execute a computer program for performing a process of creating and transmitting an email. However, the present invention is not limited thereto. The process of creating and transmitting an email need not be performed by executing a computer program if a hardware component such as ASIC (Application Specific Integrated Circuit) or FPGA (Field Programmable Gate Array) is configured to perform the same process.

The above embodiment describes a built-in type sample analyzer. However, the present invention is not limited thereto. For example, the sample analyzer may be formed with an analyzer body and a data processing apparatus (PC) connected to the analyzer body.

The above embodiment describes a case where the sample analyzer 1 is a blood coagulation measuring apparatus. However, the present invention is not limited thereto. For example, the sample analyzer may be a sample analyzer for clinical use such as a blood cell counter, biochemical analyzer, immune analyzer, or a urine analyzer, or may be a sample analyzer for industrial use.

As described above, the sample analyzer of the present invention is useful for sample analysis.

## Claims

1. A sample analyzer comprising:
a measurement section for performing measurement of a sample;
a display for displaying an analysis result that is obtained based on the measurement of the sample performed by the measurement section;
a controller for receiving a transmission instruction to transmit a screen displayed by the display; and
a transmitter for transmitting, to an external destination, image data of the screen for which the controller has received the transmission instruction, together with identification information for identifying the sample analyzer or a facility where the sample analyzer is installed.

2. The sample analyzer of claim 1, wherein
the transmitter is configured to transmit an email to which the image data is attached.

3. The sample analyzer of claim 2, wherein
the identification information is transmitted as a part of source information which indicates the source of the email.

4. The sample analyzer of claim 2, wherein
the subject of the email contains the model name of the sample analyzer.

5. The sample analyzer of claim 2, wherein
the sample analyzer does not have a function of receiving an incoming email.

6. The sample analyzer of claim 1, wherein
the identification information contains an address for linking to information that contains contact information about the facility where the sample analyzer is installed.

7. The sample analyzer of claim 1, wherein
the controller is configured to obtain status information about a status of the sample analyzer, and
the transmitter is configured to transmit the status information together with the image data and the identification information.

8. The sample analyzer of claim 1, wherein
the display comprises a touch panel type input section, and
the controller is configured to receive the transmission instruction, which is provided via the input section.

9. The sample analyzer of claim 8, wherein
the controller is configured to cause, if the controller has received a predetermined input via the input section while a screen to be transmitted is displayed by the display, the display to switch the displayed screen to a reception screen for receiving the transmission instruction, and to receive the transmission instruction via the reception screen.

10. The sample analyzer of claim 9, wherein
the reception screen includes a button for receiving the transmission instruction and a button for receiving an instruction different from the transmission instruction.

11. The sample analyzer of claim 9, wherein
the controller is configured to display a button for receiving the predetermined input, at a predetermined position on the display regardless of contents of the displayed screen.

12. The sample analyzer of claim 9, wherein
the controller is configured to store image data of the screen to be transmitted if the controller has received the predetermined input via the input section while the screen to be transmitted is displayed by the display, and to cause the display to switch the displayed screen to the reception screen for receiving the transmission instruction, and
the transmitter is configured to transmit the image data stored in the controller when the controller has received the transmission instruction.

13. The sample analyzer of claim 12, wherein
the controller is configured to delete, after receiving the predetermined input via the input section and storing the image data, the image data stored in the controller and store image data of another screen if the controller has received the predetermined input via the input section again while the other screen is displayed by the display.

14. A method for controlling a sample analyzer which comprises a measurement section for performing measurement of a sample, a display for displaying an analysis result that is obtained based on the measurement of the sample performed by the measurement section, a transmitter for transmitting information to an external destination, and a controller, the method comprising:
receiving, by the controller, a transmission instruction to transmit a screen displayed by the display; and
transmitting, to an external destination by the transmitter, image data of the screen for which the controller has received the transmission instruction, together with identification information for identifying the sample analyzer or a facility where the sample analyzer is installed.

15. A computer program product executable by a controller of a sample analyzer which comprises a measurement section for performing measurement of a sample, a display for displaying an analysis result that is obtained based on the measurement of the sample performed by the measurement section, a transmitter for transmitting information to an external destination, and the controller, the computer program product comprising:
a computer readable medium for storing instructions enabling a processor to carry out operations comprising:
receiving, by the controller, a transmission instruction to transmit a screen displayed by the display; and
transmitting, to an external destination by the transmitter, image data of the screen for which the controller has received the transmission instruction, together with identification information for identifying the sample analyzer or a facility where the sample analyzer is installed.
